(19) Europäisches Patentamt
European Patent Office
Office européen des brevets

(11) Veröffentlichungsnummer : **0 109 381**
**B1**

(12) # EUROPÄISCHE PATENTSCHRIFT

(45) Veröffentlichungstag der Patentschrift : **30.07.86**

(51) Int. Cl.⁴ : **C 07 D409/12**, A 61 K 31/41// C07D333/38, C07D333/28

(21) Anmeldenummer : **83890183.3**

(22) Anmeldetag : **18.10.83**

(54) **Thiophen-2-carbonsäurederivate und Verfahren zu deren Herstellung.**

(30) Priorität : **05.11.82 AT 4041/82**

(43) Veröffentlichungstag der Anmeldung : **23.05.84 Patentblatt 84/21**

(45) Bekanntmachung des Hinweises auf die Patenterteilung : **30.07.86 Patentblatt 86/31**

(84) Benannte Vertragsstaaten : **BE CH DE FR GB IT LI LU NL SE**

(56) Entgegenhaltungen :
**DE-A- 1 940 388**
**DE-A- 2 063 857**

(73) Patentinhaber : **Laevosan-Gesellschaft m.b.H.
Estermannstrasse 17
A-4020 Linz (AT)**

(72) Erfinder : **Binder, Dieter, Prof. Dr.
Sieveringerstrasse 207
A-1190 Wien (AT)**
Erfinder : **Noe, Christian, Mag.pharm.Dipl.Ing.Dr.
Larochegasse 20
A-1130 Wien (AT)**

**EP 0 109 381 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue therapeutisch wertvolle Thiophen-2-carbonsäurederivate und ein Verfahren zu deren Herstellung.

Insbesondere betrifft die Erfindung neue Thiophen-2-carbonsäurederivate der allgemeinen Formel

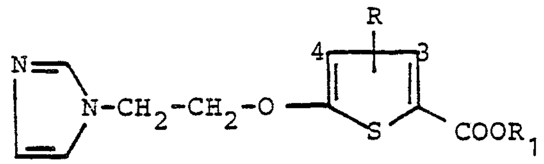

(I)

worin R in Stellung 3 oder 4 Wasserstoff, Methyl, Chlor oder Brom und $R_1$ Wasserstoff oder $C_1$-$C_4$-Alkyl bedeuten, und ihre pharmazeutisch verträglichen Säure- oder Basenadditionssalze.

Die neuen Verbindungen der Formel (I) zeigen eine starke Hemmungswirkung auf die Thromboxansynthetase ohne signifikante Hemmung der Wirkung der Prostacyclinsynthetase- oder Cyclooxigenaseenzyme aus Thrombozytenmikrosomen, d. h. die neuen Verbindungen der Formel (I) hemmen die Umwandlung von Prostaglandin-$H_2$ zu Thromboxan-$B_2$ über Thromboxan $A_2$,das ein instabiles Zwischenprodukt ist und das bekanntlich die irreversible Plättchenaggregation induziert und glatte Muskeln, insbesondere die der Blutgefäße, kontrahiert. Diese Tatsache zeigt, daß die Verbindungen der Formel (I) gemäß der Erfindung die Biosynthese von Thromboxan $A_2$ hemmen und demgemäß zur Behandlung von Krankheiten geeignet sind, die durch Thromboxan $A_2$ verursacht werden, wie z. B. Entzündung, Hypertonie, Thrombus, Gehirnschlag, Asthma, Angina pectoris, ischämische Herzleiden, ischämische Anfälle, Migräne und vaskuläre Komplikationen von Diabetes.

Von den Verbindungen der Formel (I) gemäß der Erfindung weisen sowohl die Ester als auch die freien Säuren eine starke Hemmungswirkung auf die Thromboxansynthetase auf.

Aus der DE-OS 1 940 388 und der DE-OS 2 063 857 sind bereits Imidazolderivate ähnlicher Struktur bekannt, welche jedoch anstelle des erfindungsgemäß eingesetzten Thiophenringes einen Phenylring oder andere Reste aufweisen.

Das erfindungsgemäße Verfahren ist dadurch gekennzeichnet, daß man

a) eine Verbindung der allgemeinen Formel

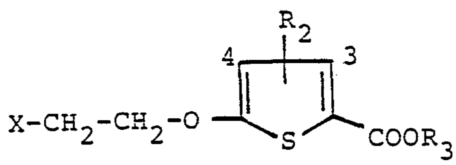

(II)

worin $R_2$ in Stellung 3 oder 4 des Thiophenkerns Wasserstoff oder Methyl und in Stellung 3 Chlor oder Brom bedeutet, X eine zum nucleophilen Austausch geeignete Abgangsgruppe, wie z. B. Halogen, vorzugsweise Chlor oder Brom, und $R_3$ $C_1$-$C_4$-Alkyl bedeutet, mit Imidazol oder einem Alkalimetallsalz hievon der Formel umsetzt,

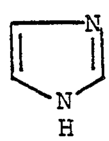

(III)

b) gegebenenfalls die so erhaltenen Verbindungen der Formel (I), worin R Wasserstoff bedeutet, mit einem zur elektrophilen Substitution geeigneten Halogenierungsmittel, wie z. B. $SO_2Hal_2$ oder N-Hal-Succinimid, wobei Hal für Chlor oder Brom steht, in Stellung 4 des Thiophenringes halogeniert

c) gegebenenfalls die erhaltenen Ester der Formel (I), worin $R_1$ $C_1$-$C_4$-Alkyl bedeutet, zu den Carbonsäuren der Formel (I) hydrolysiert und gegebenenfalls die erhaltenen Verbindungen der Formel (I) in ihre Basen- oder Säureadditionssalze überführt.

Die Reaktion wird üblicherweise durch Zugabe von 1 Äquivalent eines Alkalimetallhydrids, z. B. Natriumhydrid, zu einer Lösung von Imidazol in einem wasserfreien inerten organischen Lösungsmittel, z. B. Dimethylformamid, Dimethylsulfoxid und Hexamethylphosphorsäuretriamid, durchgeführt. Zur Vervollständigung der Salzbildung wird noch 1 h auf 90 °C erhitzt, sobald die Zugabe des Alkalimetallhydrids abgeschlossen ist. Die Lösung wird dann abgekühlt und das Halogenid der Formel (II) zugesetzt, vorzugsweise in äquivalenten Mengen oder in geringem Überschuß, gelöst im gleichen Lösungsmittel.

Der Ablauf der Reaktion bis zum Abschluß kann bei Zimmertemperatur durchgeführt werden, jedoch wird es im allgemeinen bevorzugt, das Reaktionsgemisch zu erwärmen, z. B. auf 70 °C, um die Reaktion zu beschleunigen. Unter diesen Bedingungen ist die Reaktion üblicherweise innerhalb von 1 h im wesentlichen abgeschlossen.

Das Reaktionsgemisch wird in konventioneller Weise aufgearbeitet, z. B. durch Entfernen des Lösungsmittels unter Vakuum, durch Lösungsmittelextraktion und Umkristallisieren.

Zur Einführung eines Halogensubstituenten in Stellung 4 des Thiophenkerns der Ester der Formel (I), worin R Wasserstoff bedeutet, verwendet man vorzugsweise Sulfurylchlorid bzw. Sulfurylbromid. Man geht am besten so vor, daß man die Ester der Formel (I) in einem halogenierten Kohlenwasserstoff, vorzugsweise Chloroform, löst und bei Temperaturen unter — 35 °C mit in Chloroform verdünntem oder auch unverdünntem Sulfurylhalogenid versetzt. Das Sulfurylhalogenid soll äquivalent mit nur geringem Überschuß eingesetzt werden. Die Reaktionslösung läßt man dann auf Raumtemperatur kommen und bis zu 15 h nachreagieren. Die Aufarbeitung erfolgt durch Versetzen mit wässeriger HCL und Äther, Abtrennen und Neutralisieren der wässerigen Phase, Extraktion des ausfallenden halogenierten Esters, am besten mit Methylenchlorid, Entfernen des Lösungsmittels im Vakuum und Reinigen des Produktes durch Umkristallisieren oder Säulenchromatographie.

Die Säuren der Formel (I) können in üblicher Weise durch mineralsäure- oder basenkatalysierte Hydrolyse der entsprechenden Ester (Formel I, $R_1 = C_1$-$C_4$-Alkyl) erhalten werden, z. B. unter Verwendung von wässerigem NaOH oder wässeriger Salzsäure.

Die Verbindungen der Formel (I) der Erfindung mit einem freien Carboxylrest oder einem freien Aminrest kann man auf übliche Weise in ihre pharmazeutisch verträglichen Salze umwandeln. Beispielsweise löst man die Verbindung der Formel (I) in ihrer freien Form in einem Lösungsmittel, z. B. einem Alkohol oder Wasser, gibt eine adäquate Menge Salzsäure oder Natriumhydroxid zu der Lösung zu, rührt die Mischung bei Raumtemperatur für einen adäquaten Zeitraum, destilliert danach das Lösungsmittel ab, kristallisiert den Rückstand um und erhält das Salz der Verbindung der Formel (I). Geeignete Beispiele für derartige pharmazeutisch verträgliche Salze sind neben dem Salzsäuresalz das Schwefelsäuresalz, das Salpetersäuresalz, das Phosphorsäuresalz, das Sulfonsäuresalz, das Benzoesäuresalz, das Bernsteinsäuresalz, das Weinsäuresalz und das Citronensäuresalz. Andererseits sind Beispiele für derartige pharmazeutisch verträgliche Basenadditionssalze neben dem Natriumsalz das Kaliumsalz, das Kalziumsalz und das Magnesiumsalz.

Bei den Salzen der Verbindungen der Formel (I) kann man die Salzform der Verbindungen auf übliche Weise in die freie Form der Verbindungen umwandeln. Beispielsweise löst man die Salzform der Verbindung der Formel (I) in Wasser, gibt danach eine adäquate Menge an Salzsäure oder Natriumhydroxid zu der Lösung, rührt die Mischung bei Raumtemperatur für einen adäquaten Zeitraum, entfernt Wasser, destilliert den Rückstand unter vermindertem Druck oder kristallisiert aus einem Lösungsmittel um und erhält die gewünschte Verbindung.

Säure- oder Basenadditionssalze der Verbindungen gemäß der Erfindung haben eine ebenso hohe Hemmungswirkung auf Thromboxansynthetase wie die entsprechenden Verbindungen mit einem freien Aminrest oder einem Säurerest.

Die Verbindungen der Formel (II) können z. B. ausgehend von den literaturbekannten Thiophen-2-carbonsäuren der Formel (IV) gemäß dem nachstehenden Syntheseschema hergestellt werden.

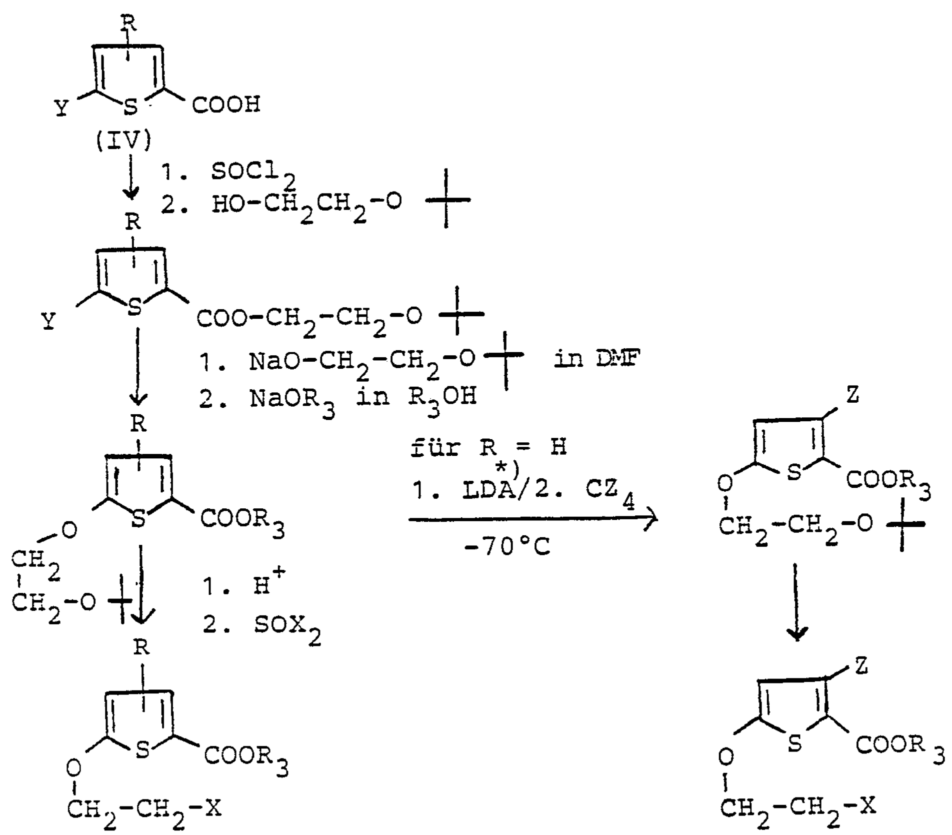


*) Lithiumdiisopropylamin

R = H oder $CH_3$, $R_3$ = $C_1$-$C_4$-Alkyl, X = Cl oder Br, Y = Cl, Br oder J, Z = Cl oder Br.
Die folgenden Beispiele sollen die vorliegende Erfindung erläutern, ohne sie einzuschränken.

Beispiel 1

5-[2-(2-Imidazolyl)-1-äthoxy]-thiophen-2-carbonsäuremethyl-ester (Formel I : R = H, $R_1$ = $CH_3$)

1,58 g 50 %ige NaH-Suspension in 33 mMol Weißöl werden zur Entfernung des Weißöls auf einer Glassinternutsche dreimal mit abs. Benzol gewaschen und nicht völlig trockengesaugt in 20 ml abs. DMF eingetragen. Zu dieser Suspension werden 2,25 g Imidazol (33 mMol, MG 68,1), gelöst in 10 ml abs. DMF, zugetropft. Danach wird bei 90 °C gerührt, bis die $H_2$-Entwicklung aufhört, was ca. 1 h benötigt. Es wird auf Raumtemperatur abgekühlt und eine Lösung von 6,6 g 5-(2-Chloräthoxy)-thiophen-2-carbonsäuremethylester (Formel II, $R_2$ = H, $R_3$ = $CH_3$, X = Cl) (30 mMol) und 0,22 g NaJ (1,5 mMol) in 10 ml abs. DMF zufließen gelassen und 1 h auf 70 °C erhitzt. Nach dem Abkühlen wird DMF abrotavapiert, der Rückstand zwischen Äther und 2n HCl verteilt, die wässerige Phase mit festem $NaHCO_3$ neutralisiert und mit $CH_2Cl_2$ extrahiert. Die $CH_2Cl_2$-Phase wird über $Na_2SO_4$ getrocknet und im Vakuum eingedampft. Ausbeute 4,0 g (53 %), Fp. 93-95 °C (Benzol) farblose Kristalle.

Auf analoge Weise wird erhalten :

5-[2-(1-Imidazolyl)-1-äthoxy]-3-methyl-thiophen-2-carbonsäure-methylester (Formel I, R = 3-$CH_3$, $R_1$ = $CH_3$)

Fp. (Äther) 79-82 °C (50 %)

5-[2-(1-Imidazolyl)-1-äthoxy]-4-methyl-thiophen-2-carbonsäure-methylester (Formel I, R = 4-Methyl, $R_1$ = $CH_3$)

Fp. (Toluol) 102-105 °C (44 %).

Das Ausgangsmaterial kann folgendermaßen hergestellt werden :

2-Chlor-3-methylthiophen

50 g (0,51 Mol) 3-Methylthiophen werden zum Sieden erhitzt und 71,43 g (0,53 Mol) $SO_2Cl_2$ während 1/2 h zugetropft. Dann wird noch 1 1/2 h am Rückfluß erhitzt und die Reaktionslösung über eine Füllkörperkolonne fraktioniert destilliert. Ausbeute 35,3 g (52 %), Kp. 152-155 °C/1 013 mbar, $n_D^{20}$ = 1,540 8.

2-Brom-3-methylthiophen

196,23 g (2 Mol) 3-Methylthiophen werden in 600 ml $CCl_4$ gelöst, die Lösung auf —10 °C abgekühlt und 319,64 g (2 Mol) $Br_2$, gelöst in 400 ml $CCl_4$, während 2 h bei dieser Temperatur zugetropft. Dann wird auf Raumtemperatur erwärmen gelassen, noch 2 h nachgerührt und die Reaktionslösung bei 22 mbar fraktioniert destilliert. Ausbeute 199 g (56 %), Kp. 66 °C/22 mbar, $n_D^{20}$ = 1,572 9.

2-Brom-5-chlor-3-methylthiophen

Zu 50 g (0,28 Mol) 2-Brom-3-methylthiophen werden 30,7 g (0,294 Mol) $SO_2Cl_2$ unter Rühren so zugetropft, daß die Temperatur 10 °C nicht übersteigt. Es wird noch 2 h bei Raumtemperatur nachgerührt und die Reaktionslösung bei 22 mbar fraktioniert destilliert. Ausbeute 37 g (62 %), Kp. 90-114 °C/22 mbar.

5-chlor-3-methylthiophen

448,53 g Zn-Staub werden in 722 ml $H_2O$ suspendiert, 280 g Essigsäure zugegeben, die Reaktionsmischung am Rückfluß erhitzt und 361,11 g 2-Brom-5-chlor-3-methylthiophen zugetropft. Nach 15 h Erhitzen am Rückfluß wird über eine Destillationsbrücke abdestilliert, die organische Phase des Destillats abgetrennt, mit gesättigter $NaHCO_3$-Lösung gewaschen und bei Normaldruck fraktioniert destilliert. Ausbeute 133 g (59 %), Kp. 153-167 °C/1 013 mbar, $n_D^{20}$ = 1,536 5.

(5-Chlor-4-methyl-2-thienyl)-methyl-keton

Eine Mischung von 20 g (0,15 Mol) 2-Chlor-3-methylthiophen und 18,6 g (0,18 Mol) Essigsäureanhyrid wird auf 70 °C erwärmt und 1,8 ml Orthophosphorsäure werden so zugetropft, daß die Reaktionstemperatur unter 75 °C bleibt. Dann wird noch 3 h bei 100 °C gerührt, mit 50 ml $H_2O$ versetzt, die

organische Phase zweimal mit 10 ml gesättigter $NaHCO_3$-Lösung extrahiert, über $Na_2SO_4$ getrocknet und im Vakuum fraktioniert destilliert. Ausbeute 15,8 g (60 %), Kp. 85-88 °C/4 mbar.

Analog wird erhalten (Reaktionstemperatur aber maximal 25 °C) :

5-(Chlor-3-methyl-2-thienyl)-methylketon, Kp. 104-110 °C/7 mbar, $n_D^{20}$ = 1,577 1 (64 %).

5-Chlor-4-methyl-thiophen-2-carbonsäure

Eine Lösung von 43,21 g (1,08 Mol) NaOH in 80 ml Wasser wird mit 160 g Eis versetzt und 28,5 g (0,40 Mol) $Cl_2$ so eingeleitet, daß die Temperatur 5 °C nicht überschreitet. Dann wird diese NaOCl-Lösung auf 55 °C erwärmt und 14,9 g (85,3 mMol) (5-Chlor-4-methyl-2-thienyl)-methyl-keton auf einmal unter Rühren zugegeben. Die Reaktionsmischung wird 1 h auf 60 °C unter Rühren erhitzt, auf Raumtemperatur abgekühlt, mit 15 g $NaHSO_3$ versetzt, mit $CH_2Cl_2$ ausgeschüttelt und mit konz. HCl angesäuert. Die farblosen Kristalle werden abgesaugt, mit Wasser gewaschen und im Vakuum bei 80 °C getrocknet. Ausbeute 11,45 g (76 %), Fp. ($CH_3OH/H_2O$) : 175-178 °C.

Analog wird erhalten :

5-Chlor-3-methyl-thiophen-2-carbonsäure, Fp. (Methanol/$H_2O$) : 149-152 °C (90 %).

5-Chlorthiophen-2-carbonsäurechlorid

40,0 g (0,256 Mol) 5-Chlorthiophen-2-carbonsäure werden mit 44,3 g (0,37 Mol) $SOCl_2$ versetzt, 4 h unter Rückfluß erhitzt, das überschüssige $SOCl_2$ abrotavapiert und der Rückstand roh in die nächste Stufe eingesetzt. Ausbeute 46,1 g, Kp. 103-105 °C/14 mbar, farbloses Öl.

Analog wird erhalten :

5-Chlor-4-methyl-thiophen-2-carbonsäurechlorid

5-Chlor-3-methyl-thiophen-2-carbonsäurechlorid

Diese werden als Rohprodukte in der nächsten Stufe eingesetzt.

5-Chlorthiophen-2-carbonsäure-[2-(2-methyl-2-propoxy)-1-äthyl]-ester

36,2 g (0,2 Mol) 5-Chlorthiophen-2-carbonsäurechlorid werden unter Rühren einer Mischung bestehend aus 26,0 g (0,22 Mol) 2-tert. Butoxyäthanol und 22,22 g (0,22 Mol) Triäthylamin in 100 ml absolutem THF bei Raumtemperatur rasch zugetropft. Dann wird 1 h bei Raumtemperatur und 10 min bei Siedetemperatur nachgerührt. Nach Abkühlen wird im Vakuum eingedampft, der Rückstand zwischen Äther und $NaHCO_3$-Lösung verteilt, die organische Phase getrocknet und im Vakuum eingedampft und schließlich im Vakuum destilliert. Rohausbeute 50,3 g (95 %), Ausbeute 42,3 g (80 %), farbloses Öl, Kp. 90-93 °C/0,007 mbar.

Analog wird erhalten :

5-Chlor-4-methyl-thiophen-2-carbonsäure-[2-(2-methyl-2-propoxy)-1-äthyl]-ester, Kp. 136 °C/0,7 mbar (84 %).

5-Chlor-3-methyl-thiophen-2-carbonsäure-[2-(2-methyl-2-propoxy)-1-äthyl]-ester, Kp. 137-139 °C/10 mbar (72 %).

5-[2-(2-Methyl-2-propoxy)-1-äthoxyl]-thiophen-2-carbonsäure-[2-(2-methyl-2-propoxy)-1-äthyl]-ester

43,95 g 57 %ige NaH-Suspension in Weißöl (1,044 Mol) werden zur Entfernung des Weißöls auf einer Glassinternutsche dreimal mit abs. Benzol gewaschen und nicht völlig trockengesaugt in kleinen Portionen einer Mischung bestehend aus 123,5 g (1,044 Mol) 2-tert.Butoxyäthanol und 300 ml frisch destilliertem abs. DMF unter Rühren zugegeben. Die Reaktionstemperatur wird dabei unter 45 °C gehalten. Nach beendeter Zugabe werden 180 g (0,069 Mol) 5-Chlorthiophen-2-carbonsäure-[2-(2-methyl-2-propoxy)-1-äthyl]-ester unter Rühren zugetropft und danach 1 h bei 65 °C gehalten. Es wird auf Raumtemperatur abgekühlt, 19,8 g (0,33 Mol) Eisessig werden zugesetzt, zwischen Äther und Natriumhydrogencarbonatlösung verteilt, die organische Phase getrocknet (Natriumsulfat) und im Vakuum eingedampft. Der ölige Rückstand wiegt 209 g (65 %) und wird ohne weitere Reinigung in der nächsten Stufe eingesetzt. Kp. 135-138 °C/0,014 mbar, farbloses Öl.

Analog werden erhalten :

3-Methyl-5-[2-(2-methyl-2-propoxy)-1-äthoxy]-thiophen-2-carbonsäure-[2-(2-methyl-2-propoxy)-1-äthyl]-ester,

Fp. (Äther/Petroläther) 43-46 °C

4-Methyl-5-[2-(2-methyl-2-propoxy)-1-äthoxy]-thiophen-2-carbonsäure-[2-(2-methyl-2-propoxy)-1-äthyl]-ester, Öl.

Beide Verbindungen werden als Rohprodukt in der nächsten Stufe eingesetzt

5-[2-(Methyl-2-propoxy)-1-äthoxy]-thiophen-2-carbonsäure-methylester

204 g roher 5-[2-(2-Methyl-2-propoxy)-1-äthoxy]-thiophen-2-carbonsäure-[2-(2-methyl-2-propoxy)-1-äthyl]-ester werden mit 11,84 g $NaOCH_3$ in 500 ml abs. Methanol 11 h am Rückflußerhitzt. Nach dem Abkühlen werden 13,5 g Eisessig zugesetzt und das Methanol bei 30 °C abrotavapiert. Der Rückstand wird zwischen Methylenchlorid und Natriumhydrogencarbonatlösung verteilt und die organische Phase getrocknet, eingedampft und im Vakuum destilliert, Ausbeute 105 g (93 %), Kp. 139-145 °C/0,14 mbar, Fp. 41-42 °C (Petroäther), farblose Kristalle.
Analog werden erhalten :

3-Methyl-5-[2-(2-methyl-2-propoxy)-1-äthoxy]-thiophen-2-carbonsäuremethylester, Kp. 136-150 °C/1,4 mbar (45 %).

4-Methyl-5-[2-(2-methyl-2-propoxy)-1-äthoxy]-thiophen-2-carbonsäuremethylester, Kp. 155-160 °C/1 mbar (33 %).

5-(2-Hydroxy-1-äthoxy)-thiophen-2-carbonsäuremethylester

105 g (0,41 Mol) 5-[2-(2-Methyl-2-propoxy)-1-äthoxy]-thiophen-2-carbonsäuremethylester werden mit 100 ml konz.Salzsäure versetzt und 15 min geschüttelt. Dann werden 100 ml Wasser zugesetzt und mit Methylenchlorid mehrmals extrahiert. Die organischen Phasen werden vereinigt, mit Natriumhydrogencarbonatlösung geschüttelt, getrocknet und eingedampft, Ausbeute 76 g (92 %).
Das Produkt kann ohne weitere Reinigung in der nächsten Stufe eingesetzt werden, Kp. 104-107 °C/0,014 mbar, farbloses Öl.
Analog werden erhalten :

3-Methyl-5-(2-hydroxy-1-äthoxy)-thiophen-2-carbonsäuremethylester, Fp. (Petroäther) 56-61 °C (98 %)

4-Methyl-5-(2-hydroxy-1-äthoxy)-thiophen-2-carbonsäuremethylester, Fp. (Benzol) 70-72 °C (90 %).

5-(2-Chlor-1-äthoxy)-thiophen-2-carbonsäuremethylester

Eine Mischung bestehend aus 45,5 g (0,225 Mol) 5-(2-Hydroxy-1-äthoxy)-thiophen-2-carbonsäuremethylester und 17,80 g (0,225 Mol) Pyridin in 20 ml abs. Chloroform werden unter mechanischem Rühren auf —40 °C gekühlt und 29,45 g (0,248 Mol) $SOCl_2$ so zugegeben, daß die Reaktionstemperatur nicht über —35 °C steigt. Nach beendeter Zugabe wird das Kühlbad entfernt und das Reaktionsgemisch erwärmen gelassen. Dann wird 1 h auf 60 °C erhitzt, abgekühlt, zwischen Äther und Wasser verteilt, die organische Phase mit 2n HCl und dann mit $NaHCO_3$-Lösung geschüttelt, getrocknet und im Vakuum eingedampft, Ausbeute 45,14 g (91 %), Fp. 54-55 °C (Petroläther), farblose Kristalle.
Analog werden erhalten :

3-Methyl-5-(2-chlor-1-äthoxy)-thiophen-2-carbonsäuremethylester, Fp. ($MeOH/H_2O$) 47-50 °C (86 %)

4-Methyl-5-(2-chlor-1-äthoxy)-thiophen-2-carbonsäuremethylester, Fp. (Petroläther) 75 °C (90 %)

## Beispiel 2

4-Chlor-5-[2-(1-imidazolyl)-1-äthoxy]-thiophen-2-carbonsäuremethylester (Formel I : R = 4-Cl, $R_1 = CH_3$)

0,15 g 5-[2-(1-Imidazolyl)-1-äthoxy]-thiophen-2-carbonsäuremethylester (Formel I : R = H, $R_1 = CH_3$) werden in 5 ml abs. Chloroform gelöst, auf —40 °C gekühlt und 0,16 g (1,19 Mol) $SO_2Cl_2$ so zugetropft, daß die Temperatur nicht über —35 °C steigt. Nach Stehenlassen über Nacht wird im Vakuum eingedampft, der Rückstand zwischen 2n HCl und Äther verteilt, die wässerige Phase mehrmals mit Äther extrahiert, mit Natriumhydrogencarbonat neutralisiert und mit Methylenchlorid mehrmals extrahiert. Die vereinigten Methylenchloridphasen werden getrocknet (Natriumsulfat) und eingedampft und der Eindampfrückstand säulenchromatographisch getrennt (Eluens : Benzol/Äthanol 9 : 1). Ausbeute 73 mg (43 %), Fp. 97-99 °C (Benzol/Äther), farblose Kristalle.

Beispiel 3

5-[2-(1-Imidazolyl)-1-äthoxy]-thiophen-2-carbonsäurehydrochlorid (Formel I : R = H, $R_1$ = H) (LG 82-4-00)

2,52 g (10 mMol) 5-[2-(1-Imidazolyl)-1-äthoxy]-thiophen-2-carbonsäuremethylester (Formel I : R = H, $R_1 = CH_3$) werden mit 0,48 g (12 mMol) NaOH in 50 ml Wasser bei Raumtemperatur gerührt, bis die Suspension klar wird (ca. 3 h). Danach wird das Reaktionsgemisch im Vakuum eingeengt, mit 10 ml 2n Salzsäure angesäuert und im Vakuum eingedampft und der Rückstand zweimal mit 20 ml Benzol zur Entfernung restlicher Feuchtigkeit abgedampft, mit 50 ml abs. Äthanol ausgekocht, vom Natriumchlorid abfiltriert und die Äthanollösung eingedampft. Der kristalline Rückstand wird in 100 ml abs. Äthanol durch Erhitzen gelöst, abgekühlt und das Produkt durch Zugabe von Äther gefällt. Ausbeute 2,18 g (80 %), Fp. 164-166 °C, farblose Kristalle.

Analog wird erhalten :

4-Chlor-5-[2-(1-imidazolyl)-1-äthoxy]-thiophen-2-carbonsäurehydrochlorid (LG 82-4-01) (Formel I : R = 4-Cl, $R_1$ = H), Fp. (Äthanol) 220 °C (Zers.), farblose Kristalle.

3-Methyl-5-[2-(1-imidazolyl)-1-äthoxy]-thiophen-2-carbonsäurehydrochlorid (Formel I : R = 3-$CH_3$, $R_1$ = H) (Verseifungstemperatur 65 °C), Fp. (Äthanol/Äther) 199-202 °C

4-Methyl-5-[2-(1-imidazolyl)-1-äthoxy]-thiophen-2-carbonsäurehydrochlorid (Formel I : R = 4$CH_3$, $R_1$ = H), Fp. 187-190 °C.

Daß es sich bei den erfindungsgemäßen Verbindungen um spezifische Inhibitoren der Thromboxansynthetase handelt, geht aus der im nachfolgenden beschriebenen in vitro-Untersuchung von zwei erfindungsgemäßen Verbindungen, nämlich LG 82-4-00 und LG 82-4-01, auf Hemmung Thromboxan (TX)-Bildung bei Blutplättchenaggregation hervor.

Bei 0,6 IU/ml thrombin-stimulierten menschlichen gewaschenen Blutplättchen betrug die $IC_{50}$ (μM) zum Hemmen der TX-Bildung (gemessen durch $TXB_2$-spezifische Radioimmunountersuchung) 1,1 für LG 82-4-00 und 1,3 für LG 82-4-01. Die beiden Verbindungen hemmten die $TXB_2$-Bildung und 1 bis 2 μg/ml Kollagen induzierten Blutplättchenaggregation um mehr als 95 %.

Weder LG 82-4-00 noch LG 82-4-01 besaßen eine Vasokonstriktions-, Proaggregations- oder antagonistische Wirksamkeit oder beeinflußten die primäre Wellen-ADP-Aggregation ($P > 0,05$).

Bei 10 μM gab es keine Hemmung der durch Arachidonsäure induzierten $PGI_2$-Bildung aus Rinderkoronararterienstücken.

Die erfindungsgemäßen Verbindungen können in Form von Tabletten oder Kapseln, welche eine Einheitsdosis der Verbindungen zusammen mit Verdünnungsmitteln, wie Maisstärke, Kalziumcarbonat, Dikalziumphosphat, Alginsäure, Lactose, Magnesiumstearat, Primogel oder Talkum enthalten, oral appliziert werden. Die Tabletten werden in herkömmlicher Weise durch Granulieren der Inhaltsstoffe und Pressen, die Kapseln durch Einfüllen in Hartgelatinekapseln geeigneter Größe hergestellt.

Die erfindungsgemäßen Verbindungen können auch parenteral beispielsweise durch intramuskuläre, intravenöse oder subkutane Injektion, appliziert werden. Für die parenterale Applikation werden sie am besten in Form einer sterilen wässerigen Lösung verwendet, welche andere gelöste Stoffe, wie tonische Mittel und Mittel zur Einstellung des pH-Wertes enthalten kann. Die Verbindungen können zu destilliertem Wasser zugesetzt werden und der pH-Wert kann unter Verwendung einer Säure, wie Citronensäure, Milchsäure oder Salzsäure, auf 3 bis 6 einstellt werden. Ausreichend gelöste Stoffe, wie Dextrose oder Salzlösung, können zugesetzt werden, um die Lösung isotonisch einzustellen. Die erhaltene Lösung kann dann sterilisiert und in sterile Glasampullen geeigneter Größe, so daß sie das gewünschte Lösungsvolumen enthalten, eingefüllt werden. Die erfindungsgemäßen Verbindungen können auch durch Infusion einer parenteralen Formulierung, wie oben beschrieben, in eine Vene appliziert werden.

Für die orale Applikation beim Menschen wird angenommen, daß der tägliche Dosierungswert einer erfindungsgemäßen Verbindung im Bereich von 0,1 bis 20 mg/kg pro Tag für einem typischen erwachsenen Patienten von 70 kg liegt. Für die parenterale Applikation wird angenommen, daß der tägliche Dosierungswert einer Verbindung der Formel (I) 0,01 bis 0,5 mg/kg pro Tag für einen typischen erwachsenen Patienten beträgt. Daher können Tabletten oder Kapseln üblicherweise 5 bis 150 mg an aktiver Verbindung für die orale Applikation bis zu dreimal am Tag enthalten. Die Dosierungseinheiten für die parenterale Applikation können 0,5 bis 35 mg an aktiver Verbindung enthalten. Eine typische Ampulle könnte daher eine 10 ml-Ampulle sein, welche 5 mg der aktiven Verbindung in 6 bis 10 ml Lösung enthält.

Selbstverständlich wird aber der Arzt in jedem Fall die tatsächliche Dosierung bestimmen, welche für den individuellen Patienten am geeignetsten ist, wobei diese mit dem Alter, der Masse und dem Ansprechen des Patienten variieren kann.

**Patentansprüche**

1. Thiophen-2-carbonsäurederivate der allgemeinen Formel

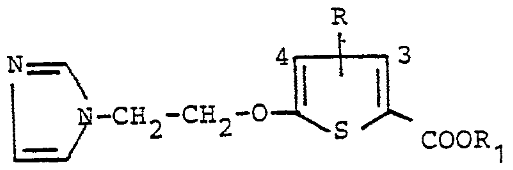

(I)

worin R in Stellung 3 oder 4 des Thiophenkerns Wasserstoff, Methyl, Chlor oder Brom und $R_1$ Wasserstoff oder $C_1$-$C_4$-Alkyl bedeuten, und ihre pharmazeutisch verträglichen Säure- oder Basenadditionssalze.

2. 5-[2-(1-Imidazolyl)-1-äthoxy]-thiophen-2-carbonsäuremethylester.

3. 5-[2-(1-Imidazolyl)-1-äthoxy]-3-methyl-thiophen-2-carbonsäuremethylester.

4. 5-[2-(1-Imidazolyl)-1-äthoxy]-4-methyl-thiophen-2-carbonsäuremethylester.

5. 4-Chlor-5-[2-(1-imidazolyl)-1-äthoxy]-thiophen-2-carbonsäuremethylester.

6. 5-[2-(1-Imidazolyl)-1-äthoxy]-thiophen-2-carbonsäurehydrochlorid.

7. 4-Chlor-5-[2-(1-imidazolyl)-1-äthoxy]-thiophen-2-carbonsäurehydrochlorid.

8. 3-Methyl-5-[2-(1-imidazolyl)-1-äthoxy]-thiophen-2-carbonsäurehydrochlorid.

9. 4-Methyl-5-[2-(1-imidazolyl)-1-äthoxy]-thiophen-2-carbonsäurehydrochlorid.

10. Verfahren zur Herstellung der Verbindungen der Formel (I), wie in Anspruch 1 definiert, und ihrer pharmazeutisch verträglichen Säure- oder Basenadditionssalze, dadurch gekennzeichnet, daß man

a) eine Verbindung der allgemeinen Formel

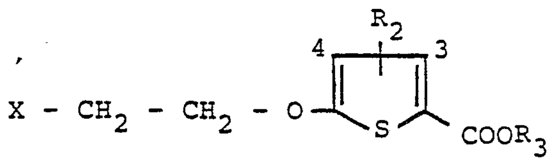

(II)

worin $R_2$ in Stellung 3 oder 4 des Thiophenringes Wasserstoff oder Methyl und in Stellung 3 Chlor oder Brom bedeutet, X eine zum nucleophilen Austausch geeignete Abgangsgruppe ist und $R_3$ $C_1$-$C_4$-Alkyl bedeutet, mit Imidazol oder einem Alkalimetallsalz hievon der Formel

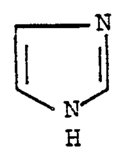

(III)

umsetzt,

b) gegebenenfalls die so erhaltenen Verbindungen der Formel (I), worin R Wasserstoff bedeutet, mit einem zur elektrophilen Substitution geeigneten Halogenierungsmittel in Stellung 4 des Thiophenringes halogeniert,

c) gegebenenfalls die erhaltenen Ester der Formel (I), worin $R_1$ $C_1$-$C_4$-Alkyl bedeutet, zu den Carbonsäuren der Formel (I) hydrolysiert und gegebenenfalls die erhaltenen Verbindungen der Formel (I) in ihre Basen- oder Säureadditionssalze überführt.

11. Verfahren nach Anspruch 10, dadurch gekennzeichnet, daß man Verbindungen (II) einsetzt, worin X Chlor oder Brom ist.

12. Verfahren nach Anspruch 10 oder 11, dadurch gekennzeichnet, daß man als Halogenierungsmittel $SO_2Hal_2$ oder N-Hal-Succinimid, wobei Hal für Chlor oder Brom steht, einsetzt.

13. Pharmazeutische Zusammensetzung, dadurch gekennzeichnet, daß sie einen wirksamen Anteil einer Verbindung der Formel (I), wie in Anspruch 1 definiert, oder eines Säure- oder Basenadditionssalzes hievon zusammen mit einem pharmazeutisch annehmbaren Träger oder Verdünnungsmittel enthält.

**Claims**

1. Derivatives of thiophene-2-carboxylic acid of the general formula

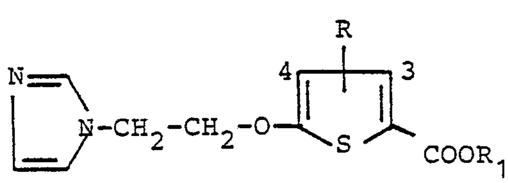

(I)

in which R is hydrogen, methyl, chlorine or bromine in position 3 or 4 of the thiophene nucleus and $R_1$ is hydrogen or $C_1$-$C_4$ alkyl, and the pharmaceutically acceptable acid or base addition salts thereof.

2. 5-[2-(1-Imidazolyl)-1-ethoxy]-thiophene-2-carboxylic acid methyl ester.
3. 5-[2-(1-Imidazolyl)-1-ethoxy]-3-methyl-thiophene-2-carboxylic acid methyl ester.
4. 5-[2-(1-Imidazolyl)-1-ethoxy]-4-methyl-thiophene-2-carboxylic acid methyl ester.
5. 4-Chloro-5-[2-(1-imidazolyl)-1-ethoxy]-thiophene-2-carboxylic acid methyl ester.
6. 5-[2-(1-Imidazolyl)-1-ethoxy]-thiophene-2-carboxylic acid hydrochloride.
7. 4-Chloro-5-[2-(1-imidazolyl)-1-ethoxy]-thiophene-2-carboxylic acid hydrochloride.
8. 3-Methyl-5-[2-(1-imidazolyl)-1-ethoxy]-thiophene-2-carboxylic acid hydrochloride.
9. 4-Methyl-5-[2-(1-imidazolyl)-1-ethoxy]-thiophene-2-carboxylic acid hydrochloride.
10. A process for preparing the compounds of formula (I), as defined in claim 1, and the pharmaceutically acceptable acid or base addition salts thereof, comprising

a) reacting a compound of general formula

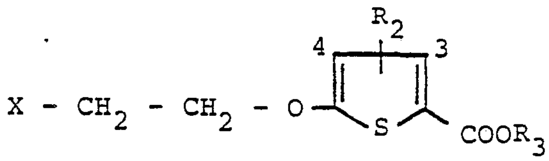

(II)

in which $R_2$ is hydrogen or methyl in position 3 or 4 of the thiophene nucleus and chlorine or bromine in position 3, X is a leaving group suitable for nucleophilic exchange and $R_3$ is $C_1$-$C_4$ alkyl, with imidazole or an alkali metal salt thereof of formula

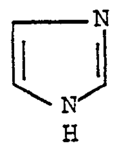

(III)

b) optionally halogenating the compounds of formula (I) such obtained, in which R is hydrogen, with a halogenating agent suitable for electrophilic substitution in position 4 of the thiophene nucleus,

c) optionally hydrolyzing the obtained esters of formula (I), in which $R_1$ is $C_1$-$C_4$ alkyl, to give the carboxylic acids of formula (I) and optionally converting the obtained compounds of formula (I) into the base or acid addition salts thereof.

11. The process of claim 10, wherein compounds (II) are used in which X is chlorine or bromine.

12. The process of claim 10 or 11, wherein $SO_2Hal_2$ or N-Hal-succinimide, in which Hal is chlorine or bromine is used as halogenating agent.

13. Pharmaceutical composition comprising an effective amount of a compound of formula (I), as defined in claim 1, or of an acid or base addition salt thereof together with a pharmaceutically acceptable carrier or diluent.

**Revendications**

1. Dérivés de l'acide thiophène-2-carboxylique de formule générale

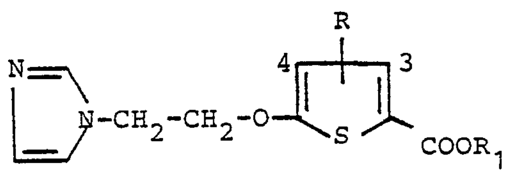

(I)

dans laquelle R représente, en position 3 ou 4 du cycle thiophène, l'hydrogène, un groupe méthyle, le chlore ou le brome, et $R_1$ représente l'hydrogène ou un groupe alkyle en $C_1$-$C_4$, et leurs sels acceptables pour l'usage pharmaceutique formés par addition avec des acides ou des bases.

2. Le 5-[2-(1-imidazolyl)-1-éthoxy]-thiophène-2-carboxylate de méthyle.
3. Le 5-[2-(1-imidazolyl)-1-éthoxy]-3-méthyl-thiophène-2-carboxylate de méthyle.
4. Le 5-[2-(1-imidazolyl)-1-éthoxy]-4-méthyl-thiophène-2-carboxylate de méthyle.
5. Le 4-chloro-5-[2-(1-imidazolyl)-1-éthoxy]-thiophène-2-carboxylate de méthyle.
6. Le chlorhydrate de l'acide 5-[2-(1-imidazolyl)-1-éthoxyl]-thiophène-2-carboxylique.
7. Le chlorhydrate de l'acide 4-chloro-5-[2(1-imidazolyl)-1-éthoxy]-thiophène-2-carboxylique.
8. Le chlorhydrate de l'acide 3-méthyl-5-[2-(1-imidazolyl)-1-éthoxy]-thiophène-2-carboxylique.
9. Le chlorhydrate de l'acide 4-méthyl-5-[2-(1-imidazolyl)-1-éthoxy]-thiophène-2-carboxylique.

10. Procédé de préparation des composés de formule I définis dans la revendication 1 et de leurs sels acceptables pour l'usage pharmaceutique formés par addition avec des acides ou des bases, caractérisé en ce que

a) on fait réagir un composé de formule générale

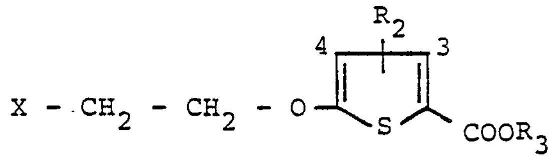

(II)

dans laquelle $R_2$ représente, en position 3 ou 4 du cycle thiophène l'hydrogène ou un groupe méthyle, et en position 3, le chlore ou le brome, X représente un groupe éliminable approprié à un échange nucléophile et $R_3$ représente un groupe alkyle en $C_1$-$C_4$, avec l'imidazole ou un sel de métal alcalin de ce composé, de formule

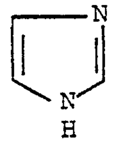

(III)

b) on halogène éventuellement les composés ainsi obtenus répondant à la formule I dans laquelle R représente l'hydrogène, en position 4 du cycle thiophène, à l'aide d'un agent halogénant convenant pour une substitution électrophile,

c) on hydrolyse le cas échéant les esters obtenus répondant à la formule I dans laquelle $R_1$ représente un groupe alkyle en $C_1$-$C_4$, en les acides carboxyliques de formule I, et le cas échéant, on convertit les composés obtenus, répondant à la formule I, en leurs sels formés par addition avec des bases ou des acides.

11. Procédé selon la revendication 10, caractérisé en ce que l'on met en œuvre des composés de formule II dans laquelle X représente le chlore ou le brome.

12. Procédé selon la revendication 10 ou 11, caractérisé en ce que l'on utilise en tant qu'agent halogénant $SO_2Hal_2$ ou N-Hal-succinimide, Hal représentant le chlore ou le brome.

13. Composition pharmaceutique caractérisée en ce qu'elle contient une proportion efficace d'un composé de formule I tel que défini dans la revendication 1 ou d'un sel d'un tel composé formé par addition avec un acide ou une base, avec un véhicule ou diluant acceptable pour l'usage pharmaceutique.